# EUROPÄISCHE PATENTANMELDUNG

(11) **EP 2 264 173 A2**
(43) Veröffentlichungstag der Anmeldung: **22.12.2010**
(21) Anmeldenummer: 10011813.2
(22) Anmeldetag: 25.10.2002
(51) Int. Cl.: C12N 15/113, A61K 48/00, A61K 31/713

(54) **Verwendung einer doppelsträngigen Ribonukleinsäure zur gezielten Hemmung der Expression eines vorgegebenen Zielgens**

(30) Priorität: 26.10.2001 DE 10155280; 29.11.2001 DE 10158411; 07.12.2001 DE 10160151; 09.01.2002 WO PCT/EP02/00151; 09.01.2002 WO PCT/EP02/00152; 02.08.2002 DE 10235620
(62) Teilanmeldung aus: 02779511.1
(71) Anmelder: Alnylam Europe AG, 95326 Kulmbach (DE)
(72) Erfinder: Limmer, Stefan, Cambridge, MA 02142 (US); Kreutzer, Roland, Cambridge, MA 02142 (US); John, Matthias, Cambridge, MA 02142 (US)
(74) Vertreter: Vossius & Partner

(57) **Zusammenfassung**

Die Erfindung umfasst die Verwendung einer doppelsträngigen Ribonukleinsäure (dsRNA) zur gezielten Hemmung der Expression eines vorgegebenen, gegenüber einem Ursprungsgen punktmutierten Zielgens in einer Zelle, wobei ein Strang S1 der dsRNA einen zum Zielgen komplementären Bereich aufweist, in welchem mindestens ein Nukleotid nicht komplementär zum Zielgen ist und die Anzahl der Nukleotide, welche nicht komplementär zum Ursprungsgen sind, um mindestens eins höher ist als die Anzahl der Nukleotide, welche nicht komplementär zum Zielgen sind.

## Beschreibung

Die Erfindung betrifft die Verwendung einer doppelsträngigen Ribonukleinsäure (dsRNA) zur gezielten Hemmung der Expression eines vorgegebenen, gegenüber einem Ursprungsgen punktmutierten Zielgens in einer Zelle. Sie betrifft weiterhin die Verwendung einer solchen Ribonukleinsäure zur Herstellung eines Medikaments, ein Medikament und ein Verfahren zur gezielten Hemmung der Expression des genannten Zielgens in einer Zelle.

Aus der DE 101 00 586 C1 ist ein Verfahren zur Hemmung der Expression eines Zielgens in einer Zelle bekannt, bei dem ein Oligoribonukleotid mit doppelsträngiger Struktur in die Zelle eingeführt wird. Ein Strang der doppelsträngigen Struktur ist dabei komplementär zum Zielgen.

Aus Elbashir, S. M. et al., Nature 411 (2001), Seiten 494 - 498 ist es bekannt, dass eine kurze dsRNA, in welcher drei Nukleotide nicht komplementär zum Zielgen sind, die Expression eines Zielgens kaum noch hemmt. Eine vollständig komplementäre dsRNA bewirkt dagegen eine effektive Hemmung der Expression des Zielgens.

Aus Holen, T. et al., Nucleic Acids Research 30 (2002), Seiten 1757 - 1766, ist es bekannt, dass eine Hemmung der Expression eines Gens durch kurze dsRNA im Wege der RNA-Interferenz auch mit dsRNAs möglich ist, deren einer Strang ein oder zwei zum Zielgen nicht komplementäre Nukleotide aufweist.

Viele Krankheiten und Entartungen von Zellen beruhen darauf, dass ein für Zellen wichtiges Gen, häufig ein Proto-Onkogen, durch eine oder wenige Punktmutationen verändert ist. Das Problem bei der Behandlung einer solchen Krankheit oder solcher Zellen mit den bisher bekannten Methoden besteht darin, dass die Inhibition der Expression des mutierten Gens häufig ebenfalls zu einer Inhibition des nicht mutierten Gens führt. Dies hat oft gravierende Nebenwirkungen zur Folge.

Aufgabe der vorliegende Erfindung ist es, die Nachteile nach dem Stand der Technik zu vermeiden. Insbesondere soll eine Verwendung einer dsRNA zur gezielten Hemmung der Expression eines gegenüber einem Ursprungsgen punktmutierten Zielgens in einer Zelle bereitgestellt werden, bei der die Expression des Ursprungsgens weitgehend unbeeinflusst bleibt. Weiterhin soll ein Medikament und ein Verfahren zur gezielten Hemmung der Expression eines vorgegebenen Zielgens sowie eine Verwendung zur Herstellung des Medikaments bereitgestellt werden. Diese Aufgabe wird durch die Merkmale der Ansprüche 1, 2, 18 und 33 gelöst. Vorteilhafte Ausgestaltungen ergeben sich aus den Merkmalen der Ansprüche 3 bis 17, 19 bis 32 und 34 bis 44.

Erfindungsgemäß ist eine Verwendung einer doppelsträngigen Ribonukleinsäure (dsRNA) zur gezielten Hemmung der Expression eines vorgegebenen, gegenüber einen Ursprungsgen punktmutierten Zielgens in einer Zelle vorgesehen, wobei ein Strang S1 der dsRNA einen zum Zielgen komplementären Bereich aufweist, in welchem mindestens ein Nukleotid nicht komplementär zum Zielgen ist und die Anzahl der Nukleotide, welche nicht komplementär zum Ursprungsgen sind, um mindestens eins höher ist als die Anzahl der Nukleotide, welche nicht komplementär zum Zielgen sind. Die Erfindung betrifft weiterhin die Verwendung einer solchen dsRNA zur Herstellung eines Medikaments zur gezielten Hemmung der Expression eines vorgegebenen, gegenüber einem Ursprungsgen punktmutierten Zielgens in einer Zelle. Ein Nukleotid ist im Sinne dieser Erfindung "komplementär" zum Zielgen oder Ursprungsgen, wenn es mit einem ihm darin in seiner Sequenzposition entsprechenden Nukleotid eine spezifische Watson-Crick-Basenpaarung ausbilden kann. Unter dem "komplementären Bereich" wird verstanden, dass die darin enthaltenen Nukleotide im Wesentlichen komplementär zum Zielgen sind. Das bedeutet, dass nicht alle Nukleotide in dem Bereich komplementär zum Zielgen sind. Die Anzahl der Nukleotide, welche nicht komplementär zum Zielgen sind, ist im niedrigsten Fall eins. Unter dem Zielgen wird im Allgemeinen der DNA-Strang der doppelsträngigen in der Zelle vorhandenen DNA verstanden, welcher komplementär zu einem bei der Transkription als Matrize dienenden DNA-Strang einschließlich aller transkribierten Bereiche ist. Es handelt sich dabei also im Allgemeinen um den Sinn-Strang. Der Strang S1 kann somit komplementär zu einem bei der Expression gebildeten RNA-Transkript oder dessen Prozessierungsprodukt, wie z.B. einer mRNA, sein. Es kann z.B. ausreichend sein, wenn der Strang S1 komplementär zu einem Teil des 3'-untranslatierten Bereichs der mRNA ist. Bei dem Zielgen kann es sich aber auch um einen Teil eines viralen Genoms handeln. Das virale Genom kann auch das Genom eines (+)-Strang-RNA-Virus, insbesondere eines Hepatitis C-Virus, sein.

Das Ursprungsgen kann jedes beliebige Gen sein, welches von dem zu hemmenden Zielgen nur durch eine oder wenige Punktmutationen abweicht. Im Allgemeinen handelt es sich dabei um ein Wildtyp-Gen. Eine dsRNA liegt vor, wenn die aus einem oder zwei Nukleinsäure-Strängen bestehende Ribonukleinsäure eine doppelsträngige Struktur aufweist. Nicht alle Nukleotide der dsRNA müssen innerhalb der dsRNA kanonische Watson-Crick-Basenpaarung aufweisen. Die maximal mögliche Zahl der Basenpaare ist die Zahl der Nukleotide in dem kürzesten in der dsRNA enthaltenden Strang. Der zum Zielgen komplementäre Bereich kann weniger als 25 aufeinander folgende Nukleotide, insbesondere 19 bis 24, bevorzugt 20 bis 24, besonders bevorzugt 21 bis 23, insbesondere 22 oder 23, Nukleotide aufweisen. Der Strang S1 kann weniger als 30, vorzugsweise weniger als 25, besonders vorzugsweise 21 bis 24, insbesondere 23, Nukleotide aufweisen. Es hat sich gezeigt, dass kurze dsRNAs besonders effizient in der Hemmung der Expression eines Zielgens sind. Diese dsRNAs werden auch als siRNAs (short interfering RNAs) bezeichnet.

Bei der gezielten Hemmung wird die Expression des Ursprungsgens weniger inhibiert als diejenige des Zielgens. Im Idealfall bleibt die Expression des Ursprungsgens weit gehend unbeeinflusst. Dazu wird gezielt eine die Expression des Zielgens nicht optimal hemmende dsRNA verwendet. So kann eine dsRNA bereitgestellt werden, welche zum Ursprungsgen so wenig komplementär ist, dass dessen Expression weit gehend unbeeinflusst bleibt. Nebenwirkungen durch Hemmung des Ursprungsgens können vermieden oder verringert werden.

Die Hemmung der Expression durch die dsRNA erfolgt vorzugsweise nach dem Prinzip der RNA-Interferenz. Das zum Zielgen nicht komplementäre Nukleotid ist bevorzugt nicht am 3'- oder am 5'-Ende des Bereichs gelegen. Idealerweise liegt das nicht komplementäre Nukleotid im mittleren Teil des Bereichs. Das Zielgen kann gegenüber dem Ursprungsgen eine oder zwei Punktmutationen aufweisen. Dann ist die erfindungsgemäße Verwendung zur gezielten Hemmung der Expression des Zielgens besonders geeignet, gezielt nur diese Expression, nicht aber diejenige des Ursprungsgens zu hemmen.

Bei einer Ausgestaltung der Erfindung ist das Ursprungsgen ein Proto-Onkogen und das Zielgen ein davon abgeleitetes Onkogen. Ein Onkogen unterscheidet sich häufig von dem ihm entsprechenden zellulären Proto-Onkogen nur durch eine einzige Punktmutation. Die Hemmung der Expression des Onkogens mit herkömmlicher dsRNA bewirkt daher üblicherweise auch eine Hemmung der Expression des entsprechenden Proto-Onkogens. Das ist häufig mit so schwer wiegenden Nebenwirkungen verbunden, dass eine Verwendung herkömmlicher dsRNA zur Hemmung des Zielgens in einem Organismus kaum möglich ist.

Die Zelle kann dabei eine Tumorzelle sein. Bei einer Ausgestaltung der Erfindung ist ein Nukleotid des Bereichs nicht komplementär zum Zielgen und zwei Nukleotide des Bereichs sind nicht komplementär zum Ursprungsgen. Bereits ein derart geringer Unterschied in der Zahl komplementärer Nukleotide kann ausreichen, um die Expression des Zielgens nahezu vollständig zu hemmen und die Expression des Ursprungsgens weitgehend unbeeinflusst zu lassen.

In einer Ausgestaltung des Verfahrens ist innerhalb der dsRNA mindestens ein Basenpaar nicht komplementär, d.h. die Nukleotide des Basenpaars sind nicht spezifisch nach Watson-Crick gepaart. Durch die Variation der Zahl der nicht komplementären Basenpaare innerhalb der dsRNA kann die Wirksamkeit der dsRNA moduliert werden. Eine reduzierte Komplementarität innerhalb der dsRNA verringert deren Stabilität in der Zelle.

Vorzugsweise weist die dsRNA zumindest an einem Ende der dsRNA einen aus 1 bis 4, insbesondere 2 oder 3, Nukleotiden gebildeten einzelsträngigen Überhang auf. Ein Ende ist dabei ein Bereich der dsRNA, in welchem ein 5'- und ein 3'-Strangende vorliegen. Eine nur aus dem Strang S1 bestehende dsRNA weist demnach eine Schleifenstruktur und nur ein Ende auf. Eine aus dem Strang S1 und einem Strang S2 gebildete dsRNA weist zwei Enden auf. Ein Ende wird dann jeweils von einem auf dem Strang S1 und einem auf dem Strang S2 liegenden Strangende gebildet. Einzelsträngige Überhänge verringern die Stabilität der dsRNA in Blut, Serum und Zellen und verstärken gleichzeitig die expressionshemmende Wirkung der dsRNA. Besonders vorteilhaft ist es, wenn die dsRNA den Überhang ausschließlich an einem, insbesondere ihrem das 3'-Ende des Strangs S1 aufweisenden, Ende aufweist. Das andere Ende ist dann bei einer zwei Enden aufweisenden dsRNA glatt, d.h. ohne Überhänge, ausgebildet. Überraschenderweise hat es sich gezeigt, dass zur Verstärkung der expressionshemmenden Wirkung der dsRNA ein Überhang an einem Ende der dsRNA ausreichend ist, ohne dabei die Stabilität in einem solchen Maße zu erniedrigen wie durch zwei Überhänge. Eine dsRNA mit nur einem Überhang hat sich sowohl in verschiedenen Zellkulturmedien als auch in Blut, Serum und Zellen als hinreichend beständig und besonders wirksam erwiesen. Die Hemmung der Expression ist besonders effektiv, wenn sich der Überhang am 3'-Ende des Strangs S1 befindet.

Vorzugsweise weist die dsRNA neben dem Strang S1 einen Strang S2 auf, d.h. sie ist aus zwei Einzelsträngen gebildet. Besonders wirksam ist die dsRNA, wenn der Strang S1 (Antisinn-Strang) eine Länge von 23 Nukleotiden, der Strang S2 eine Länge von 21 Nukleotiden und das 3'-Ende des Strangs S1 einen aus zwei Nukleotiden gebildeten einzelsträngigen Überhang aufweist. Das am 5'-Ende des Strangs S1 gelegene Ende der dsRNA ist dabei glatt ausgebildet.

Der Strang S1 kann zum primären oder prozessierten RNA-Transkript des Zielgens komplementär sein. Die dsRNA kann in einer Zubereitung vorliegen, die zur Inhalation, oralen Aufnahme, Infusion und Injektion, insbesondere zur intravenösen, intraperitonealen oder intratumoralen Infusion oder Injektion, geeignet ist. Die Zubereitung kann dabei, insbesondere ausschließlich, aus einem physiologisch verträglichen Lösungsmittel, vorzugsweise einer physiologischen Kochsalzlösung oder einem physiologisch verträglichen Puffer, und der dsRNA bestehen. Der physiologisch verträgliche Puffer kann eine phosphatgepufferte Salzlösung sein.. Es hat sich nämlich überraschenderweise herausgestellt, dass eine lediglich in einem solchen Lösungsmittel oder einem solchen Puffer gelöste und verabreichte dsRNA von der Zelle aufgenommen wird und die Expression des Zielgens hemmt, ohne dass die dsRNA dazu in einem besonderen Vehikel verpackt sein muss.

Vorzugsweise liegt die dsRNA in einer physiologisch verträglichen Lösung, insbesondere einem physiologisch verträglichen Puffer oder einer physiologischen Kochsalzlösung, oder von einer micellaren Struktur, vorzugsweise einem Liposom, einem Virus-Kapsid, einem Kapsoid oder einer polymeren Nano- oder Mikrokapsel umschlossen oder an einer polymeren Nano- oder Mikrokapsel gebunden vor. Der physiologisch verträgliche Puffer kann eine phosphatgepufferte Salzlösung sein. Eine micellare Struktur, ein Virus-Kapsid, ein Kapsoid oder eine polymere Nano- oder Mikrokapsel kann die Aufnahme der dsRNA in infizierte Zellen erleichtern. Die polymere Nano- oder Mikrokapsel besteht aus mindestens einem biologisch abbaubaren Polymer, z.B. Polybutylcyanoacrylat. Die polymere Nano- oder Mikrokapsel kann darin enthaltene oder daran gebundene dsRNA im Körper transportieren und freisetzen. Die dsRNA kann oral, mittels Inhalation, Infusion oder Injektion, insbesondere intravenöser, intraperitonealer oder intratumoraler Infusion oder Injektion, verabreicht werden. Vorzugsweise wird die dsRNA in einer Dosierung von höchstens 5 mg, insbesondere höchstens 2,5 mg, bevorzugt höchstens 200 µg, besonders bevorzugt höchstens 100 µg, vorzugsweise höchstens 50 µg, insbesondere höchstens 25 µg, pro kg Körpergewicht und Tag verwendet. Es hat es sich nämlich gezeigt, dass die dsRNA bereits in dieser Dosierung eine ausgezeichnete Effektivität in der Hemmung der Expression des vorgegebenen Zielgens aufweist.

Die Erfindung betrifft weiterhin ein Medikament zur gezielten Hemmung der Expression eines vorgegebenen, gegenüber einem Ursprungsgen punktmutierten Zielgens in einer Zelle, wobei das Medikament eine doppelsträngige Ribonukleinsäure(dsRNA) enthält, wobei ein Strang S1 der dsRNA einen zum Zielgen komplementären Bereich aufweist, in welchem mindestens ein Nukleotid nicht komplementär zum Zielgen ist und die Anzahl der Nukleotide, welche nicht komplementär zum Ursprungsgen sind, um mindestens eins höher ist als die Anzahl der Nukleotide, welche nicht komplementär zum Zielgen sind. Vorzugsweise liegt das Medikament in mindestens einer Verabreichungseinheit vor, welche die dsRNA in einer Menge enthält, die eine Dosierung von höchstens 5 mg, insbesondere höchstens 2,5 mg, bevorzugt höchstens 200 µg, besonders bevorzugt höchstens 100 µg, vorzugsweise höchstens 50 µg, insbesondere höchstens 25 µg, pro kg Körpergewicht und Tag ermöglicht. Die Verabreichungseinheit kann für eine einmalige Verabreichung bzw. Einnahme pro Tag konzipiert sein. Dann ist die gesamte Tagesdosis in einer Verabreichungseinheit enthalten. Ist die Verabreichungseinheit für eine mehrmalige Verabreichung bzw. Einnahme pro Tag konzipiert, so ist die dsRNA darin in einer entsprechend geringeren das Erreichen der Tagesdosis ermöglichenden Menge enthalten. Die Verabreichungseinheit kann auch für eine einzige Verabreichung bzw. Einnahme für mehrere Tage konzipiert sein, z. B. indem die dsRNA über mehrere Tage freigesetzt wird. Die Verabreichungseinheit enthält dann ein entsprechend Mehrfaches der Tagesdosis.

Erfindungsgemäß ist weiterhin ein Verfahren zur gezielten Hemmung der Expression eines vorgegebenen, gegenüber einem Ursprungsgen punktmutierten Zielgens in einer Zelle vorgesehen, wobei eine doppelsträngige Ribonukleinsäure(dsRNA) in die Zelle eingeführt wird und ein Strang S1 der dsRNA einem zum Zielgen komplementären Bereich aufweist, in welchem mindestens ein Nukleotid nicht komplementär zum Zielgen ist und die Anzahl der Nukleotide, welche nicht komplementär zum Ursprungsgen sind, um mindestens eins höher ist als die Anzahl der Nukleotide, welche nicht komplementär zum Zielgen sind. Wegen der weiteren vorteilhaften Ausgestaltungen des erfindungsgemäßen Medikaments und des erfindungsgemäßen Verfahrens wird auf die vorangegangenen Ausführungen verwiesen.

Die Erfindung wird nachfolgend anhand der Zeichnungen beispielhaft erläutert. Es zeigen:
- Fig. 1: eine grafische Darstellung der Hemmung der Expres- sion eines HCV-Luziferase-Fusionsproteins durch dsRNAs, welche in unterschiedlichem Maße komplemen- tär zu einer Sequenz eines Zielgens sind und
- Fig. 2: eine grafische Darstellung der Hemmung der Expres- sion eines HCV-Luziferase-Fusionsproteins durch dsRNAs, welche in unterschiedlichem Maße komplemen- tär zu einer Sequenz eines Zielgens sind und teil- weise aus nicht vollständig zueinander komplementä- ren RNA-Strängen gebildet sind.

Zu Herstellung eines Reportersystems wurde eine 26 Nukleotide lange Sequenz einer als Zielgen dienenden, dem 3'-nichttranslatierten Bereich einer HCV-RNA entsprechenden cDNA-Sequenz mit dem offenen Leserahmen des Luziferase-Gens aus dem Expressionsvektor pGL3 fusioniert. Der Expressionsvektor pGL3 stammt von der Firma Promega und ist unter der Gene Accession Number U47296 beim National Center for Biotechnology Information (NCBI), National Library of Medicine, Building 38A, Bethesda, MD 20894, USA, registriert worden. Als Luziferase-Gen sind die Nukleotide 280 bis 1932 verwendet worden. Die 26 Nukleotide lange Sequenz ist eine in sehr vielen HCV-Genomen und deren Subtypen vorkommende hoch konservierte Sequenz. Bei dem unter der Gene Accession Number D89815 beim NCBI registrierten HCV-Genom entsprechen die 26 Nukleotide den Nukleotiden 9531 bis 9556. Sie weisen die folgende Sequenz auf:
5'-gtcacggct agctgtgaaa ggtccgt-3' (SEQ ID NO: 1).

Das resultierende Fusions-Gen ist als BamHI/NotI-DNA-Fragment in das eukaryotische Expressionsplasmid pcDNA 3.1 (+) von der Firma Invitrogen GmbH, Technologiepark Karlsruhe, Emmy-Noether Strasse 10, D-76131 Karlsruhe, Katalog Nr. V790-20, kloniert worden. Das resultierende Plasmid wird als p8 bezeichnet.

Als Kontrolle für die Transfektionseffizienz ist das Plasmid pCMVβGal der Firma Clontech, Gene Accession Number U13186, NCBI, verwendet worden. Dieses Plasmid kodiert für das Enzym β-Galaktosidase und bewirkt dessen Expression.

Das das Fusions-Gen enthaltende Plasmid, das zur Kontrolle dienende Plasmid und unterschiedliche dsRNAs sind gemeinsam durch Transfektion in Zellen der Leberzelllinie HuH-7 (JCRB0403, Japanese Collection of Research Bioresources Cell Bank, National Institute of Health Sciences, Kamiyoga 1-18-1, Setagaya-ku, Tokyo 158, Japan) eingeführt worden. Die durch die dsRNAs herbeigeführte Hemmung der Expression des Luziferase-Gens ist im Verhältnis zur Expression des β-Galaktosidase-Gens bestimmt worden.

Die eingesetzten dsRNAs weisen folgende, im Sequenzprotokoll mit SEQ ID NO:2 bis SEQ ID NO:13 bezeichneten Sequenzen auf:
HCV1+2, deren Strang S1 vollständig komplementär zu der HCV-Sequenz in dem fusionierten HCV-Luziferase-Gen ist:

| | |
|---|---|
| S2: | 5'- ACG GCU AGC UGU GAA AGG UCC GU-3' (SEQ ID NO:2) |
| S1: | 3'-AG UGC CGA UCG ACA CUU UCC AGG -5' (SEQ ID NO:3) |

HCV3+4, welche weder zu der HCV- noch zu der Luziferase-Sequenz in dem fusionierten HCV-Luziferase-Gen komplementär ist und als Negativkontrolle dient:

| | |
|---|---|
| S2: | 5'- AGA CAG UCG ACU UCA GCC U GG-3' (SEQ ID NO:12) |
| S1: | 3'-GG UCU GUC AGC UGA AGU CGG A -5' (SEQ ID NO:13) |

HCV5+6, deren Strang S1 abgesehen von dem durch Fettdruck hervorgehobenen Nukleotid komplementär zu der HCV-Sequenz in dem fusionierten HCV-Luziferase-Gen ist:

| | |
|---|---|
| S2: | 5'- ACG GCU AGC UGU GAA **U**GG UCC GU-3' (SEQ ID NO:6) |
| S1: | 3'-AG UGC CGA UCG ACA CUU **A**CC AGG -5' (SEQ ID NO:7) |

HCV7+8, deren Strang S1 abgesehen von den zwei durch Fettdruck hervorgehobenen Nukleotiden komplementär zu der HCV-Sequenz in dem fusionierten HCV-Luziferase-Gen ist:

| | |
|---|---|
| S2: | 5'- ACG GC**A** AGC UGU GAA **U**GG UCC GU-3' (SEQ ID NO:8) |
| S1: | 3'-AG UGC CGU UCG ACA CUU **A**CC AGG -5' (SEQ ID NO:9) |

Lucl+2, deren Strang S1 vollständig komplementär zu einer Luziferase-Sequenz in dem fusionierten HCV-Luziferase-Gen ist und als Positivkontrolle dient:

| | |
|---|---|
| S2: | 5'- CGU UAU UUA UCG GAG UUG CAG UU-3' (SEQ ID NO: 10) |
| S1: | 3'-GC GCA AUA AAU AGC CUC AAC GUC -5' (SEQ ID NO: 11) |

K3s+K3as, welche weder zu der HCV- noch zu einer Luziferase-Sequenz in dem fusionierten HCV-Luziferase-Gen komplementär ist und als Negativkontrolle dient:

| | |
|---|---|
| S2: | 5'- G AUG AGG AUC GUU UCG CAU GA-3' (SEQ ID NO: 4) |
| S1: | 3'-UCC UAC UCC UAG CAA AGC GUA -5' (SEQ ID NO: 5) |

HCV5+2, deren Strang S1 vollständig komplementär zu der HCV-Sequenz ist und deren Strang S2 abgesehen von dem durch Fettdruck hervorgehobenen Nukleotid komplementär zu der HCV-Sequenz in dem fusionierten HCV-Luziferase-Gen ist:

| | |
|---|---|
| S2: | 5'- ACG GCU AGC UGU GAA UGG UCC GU-3' (SEQ ID NO:6) |
| S1: | 3'-AG UGC CGA UCG ACA CUU UCC AGG -5' (SEQ ID NO:3) |

HCV1+6, deren Strang S2 vollständig komplementär zu der HCV-Sequenz ist und deren Strang S1 abgesehen von dem durch Fettdruck hervorgehobenen Nukleotid komplementär zu der HCV-Sequenz in dem fusionierten HCV-Luziferase-Gen ist:

| | |
|---|---|
| S2: | 5'- ACG GCU AGC UGU GAA AGG UCC GU-3' (SEQ ID NO:2) |
| S1: | 3'-AG UGC CGA UCG ACA CUU **A**CC AGG -5' (SEQ ID NO:7) |

HuH-7-Zellen sind in DMEM mit 10 % FCS kultiviert worden. Zur Vorbereitung einer Transfektion wurden 2 x 10E4 Zellen pro Vertiefung einer 96-Well-Zellkulturplatte ausgesät. Die Zellen sind 24 Stunden nach der Aussaat mittels je 110 µl Transfektionsmedium pro Vertiefung der 96-Well-Zellkulturplatte transfiziert und in diesem Gesamtvolumen weiter kultiviert worden. Jede Transfektion ist dreifach durchgeführt worden.

Dazu sind zunächst 3 µg des Plasmids pCMVβGal mit 1 µg des Plasmids p8 gemischt worden. Das Transfektionsmedium enthielt je Vertiefung 0,25 µg des Gemischs der Plasmide und 200, 100, 50, 25, 12,5 oder 0 nmol/l einer der genannten dsRNAs.

Zur Transfektion wurde "Gene Porter 2" der Firma PEQLAB Biotechnologie GmbH, Carl-Thiersch-Str. 2 b, D-91052 Erlangen, Katalog Nummer 13-T202007 gemäß Herstellervorschrift eingesetzt.

Anschließend sind die Zellen bei 37°C und 5% CO₂ inkubiert worden. Einen Tag nach der Transfektion sind pro Vertiefung 35 µl frisches Medium zugegeben und die Zellen für weitere 24 h inkubiert worden.

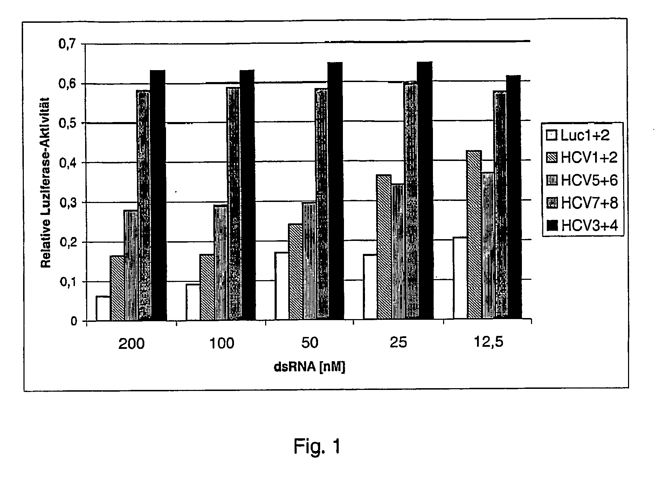

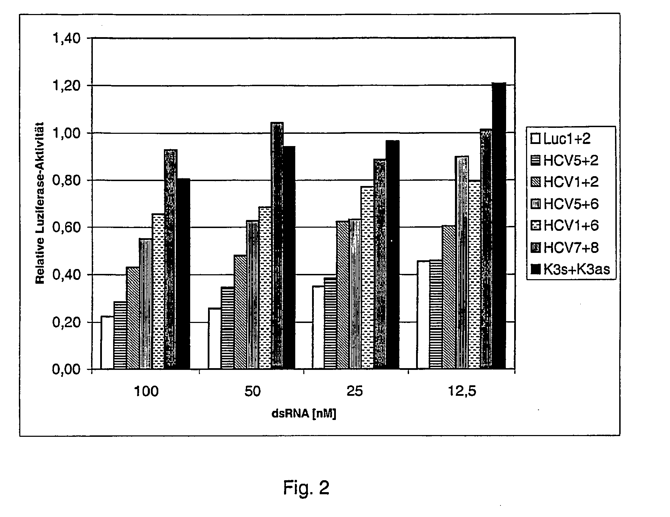

Der Effekt der eingesetzten dsRNAs wurde durch Quantifizierung der exprimierten β-Galactosidase mittels "Galacto-Star" der Firma Tropix, 47 Wiggins Avenue, Bedford, MA 01730, USA Katalog-Nummer BM100S, und der Effekt der exprimierten Luziferase mittels "Luciferase" der Firma Tropix, Katalog-Nummer BC100L, durch Chemolumineszenz-Reaktion ermittelt. Dazu wurden Lysate der Zellen gemäß den Herstellervorschriften hergestellt und davon für den Nachweis der β-Galactosidase je 2 µl pro Analyse und für den Nachweis der Luziferase je 5 µl pro Analyse eingesetzt. Die Messung der Chemolumineszenz erfolgte in einem Sirius-Luminometer der Firma Berthold Detection Systems GmbH, Bleichstrasse 56-68, D-75173 Pforzheim, Deutschland. Die relative Aktivität der Luziferase als Maß für die Expression wird ermittelt, indem jeweils der für Luziferase ermittelte Wert der Chemolumineszenz durch den für β-Galactosidase ermittelten Wert dividiert wird. Für jeweils 3 so ermittelte Werte wird ein Mittelwert berechnet. Der Mittelwert für ohne dsRNA transfizierte Zellen wird willkürlich als 1,0 definiert. Die anderen Mittelwerte sind dazu ins Verhältnis gesetzt und in den Figuren 1 und 2 grafisch dargestellt.

Luc1+2 (Positivkontrolle) führte zur stärksten Hemmung der Luziferaseaktivität (Fig. 1 und 2). In Gegenwart von HCV1+2, welches vollständig komplementär zur Zielsequenz für das Reporterplasmid war, ist ebenfalls eine deutliche Reduktion der Luziferaseaktivität erkennbar (Fig. 1 und 2). Mit abnehmender Konzentration von HCV1+2 stieg die Luziferaseaktivität an. Das in einem Nukleotid nicht zur Zielsequenz komplementäre Oligonukleotid HCV5+6 ist ähnlich effizient in der Luziferasehemmung wie HCV1+2, insbesondere bei niedrigen Konzentrationen (Fig. 1). Für die Spezifität dieser dsRNA bedeutet das, dass es nicht ausreicht, wenn die dsRNA zum Zielgen komplementär, aber zum Ursprungsgen mit einem Nukleotid nicht komplementär ist, um die Expression des Zielgens spezifisch gegenüber der Expression des Ursprungsgens zu hemmen.

HCV7+8 hemmt die Expression der Luziferase sowohl bei hohen als auch bei niedrigen Konzentrationen nur im Maße der Negativkontrollen HCV3+4 und K3S+K3AS (Fig. 1 und 2). Die geringe Hemmung der Luziferaseaktivität ist als unspezifischer Effekt zu erachten. Für die Spezifität dieser dsRNA bedeutet das, dass es ausreicht, wenn die dsRNA zu dem Zielgen komplementär oder nur mit einem Nukleotid nicht komplementär, aber zum Ursprungsgen in zwei Nukleotiden nicht komplementär ist, um die Expression des Zielgens spezifisch gegenüber der Expression des Ursprungsgens zu hemmen.

In HCV5+2 ist ein Nukleotid im Sinn-Strang S2 nicht komplementär zum Antisinn-Strang S1, wobei der Antisinn-Strang S1 vollständig komplementär zum Zielgen ist. Diese dsRNA ist so effizient wie LUC1+2 und HCV1+2 (Fig. 1 + 2). Das ist überraschend, da eine um ein Basenpaar reduzierte Komplementarität innerhalb der dsRNA eine geringere Stabilität der dsRNA in der Zelle und deshalb eine geringere Effizienz erwarten ließ.

In HCV6+1 ist ein Nukleotid im Antisinn-Strang S1 nicht komplementär zum Sinn-Strang S2, wobei der Antisinn-Strang S1 auch mit einem Nukleotid nicht komplementär zum Zielgen ist. HCV6+1 hemmt die Expression weniger effizient als HCV5+6, aber effizienter als HCV7+8 (Fig. 2). Für die Spezifität und Effizienz der expressionshemmenden Wirkung der dsRNA kommt es somit mehr auf die Sequenz des Antisinn-Strangs S1 als diejenige des Sinn-Strangs S2 an.

Die als Negativkontrolle dienenden HCV3+4 (Fig. 1) und K3S+K3AS (Fig. 2) führten zu keiner bzw. einer geringen Hemmung der Luziferase-Aktivität. Die geringe Hemmung ist unspezifisch, da sie nicht von der eingesetzten Konzentration der dsRNAs abhängt.

Die Daten zeigen, dass mindestens zwei nicht zu einem Ursprungsgen komplementäre Nukleotide im Antisinn-Strang einer dsRNA notwendig sind, um eine Inhibition der Expression des Ursprungsgens zu verhindern. Weiterhin zeigen die Daten, dass eine Modulation der Wirksamkeit der Hemmung der Expression durch eine dsRNA möglich ist, indem das Maß der Komplementarität der die dsRNA bildenden Einzelstränge verringert wird.

Die Erfindung umfasst ferner die folgenden Ausführungsformen:
1. Verwendung einer doppelsträngigen Ribonukleinsäure (dsRNA) zur gezielten Hemmung der Expression eines vorgegebenen, gegenüber einem Ursprungsgen punktmutierten Zielgens in einer Zelle, wobei ein Strang S1 der dsRNA einen zum Zielgen komplementären Bereich aufweist, in welchem mindestens ein Nukleotid nicht komplementär zum Zielgen ist und die Anzahl der Nukleotide, welche nicht komplementär zum Ursprungsgen sind, um mindestens eins höher ist als die Anzahl der Nukleotide, welche nicht komplementär zum Zielgen sind.
2. Verwendung einer doppelsträngigen Ribonukleinsäure (dsRNA) zur Herstellung eines Medikaments zur gezielten Hemmung der Expression eines vorgegebenen, gegenüber einem Ursprungsgen punktmutierten Zielgens in einer Zelle, wobei ein Strang S1 der dsRNA einen zum Zielgen komplementären Bereich aufweist, in welchem mindestens ein Nukleotid nicht komplementär zum Zielgen ist und die Anzahl der Nukleotide, welche nicht komplementär zum Ursprungsgen sind, um mindestens eins höher ist als die Anzahl der Nukleotide, welche nicht komplementär zum Zielgen sind.
3. Verwendung nach Ausführungsform 1 oder 2, wobei das zum Zielgen nicht komplementäre Nukleotid nicht am 3'- oder am 5'-Ende des Bereichs gelegen ist.
4. Verwendung nach einer der vorhergehenden Ausführungsformen, wobei das Zielgen gegenüber dem Ursprungsgen eine oder zwei Punktmutationen aufweist.
5. Verwendung nach einer der vorhergehenden Ausführungsformen, wobei das Ursprungsgen ein Proto-Onkogen und das Zielgen ein davon abgeleitetes Onkogen ist.
6. Verwendung nach einer der vorhergehenden Ausführungsformen, wobei die Zelle eine Tumorzelle ist.
7. Verwendung nach einer der vorhergehenden Ausführungsformen, wobei ein Nukleotid des Bereichs nicht komplementär zum Zielgen ist und zwei Nukleotide des Bereichs nicht komplementär zum Ursprungsgen sind.
8. Verwendung nach einer der vorhergehenden Ausführungsformen, wobei innerhalb der dsRNA mindestens ein Basenpaar nicht komplementär ist.
9. Verwendung nach einer der vorhergehenden Ausführungsformen, wobei die dsRNA zumindest an einem Ende der dsRNA einen aus 1 bis 4, insbesondere 2 oder 3, Nukleotiden gebildeten einzelsträngigen Überhang aufweist.
10. Verwendung nach Ausführungsformen 9, wobei die dsRNA den Überhang ausschließlich an einem, insbesondere ihrem das 3'-Ende des Strangs S1 aufweisenden, Ende aufweist.
11. Verwendung nach einer der vorhergehenden Ausführungsformen, wobei die dsRNA neben dem Strang S1 einen Strang S2 aufweist und der Strang S1 eine Länge von 23 Nukleotiden, der Strang S2 eine Länge von 21 Nukleotiden und das 3'-Ende des Strangs S1 einen aus zwei Nukleotiden gebildeten einzelsträngigen Überhang aufweist, während das am 5'-Ende des Strangs S1 gelegene Ende der dsRNA glatt ausgebildet ist.
12. Verwendung nach einer der vorhergehenden Ausführungsformen, wobei der Strang S1 zum primären oder prozessierten RNA-Transkript des Zielgens komplementär ist.
13. Verwendung nach einer der vorhergehenden Ausführungsformen, wobei die dsRNA in einer Zubereitung vorliegt, die zur Inhalation, oralen Aufnahme, Infusion oder Injektion, insbesondere zur intravenösen, intraperitonealen oder intratumoralen Infusion oder Injektion, geeignet ist.
14. Verwendung nach Ausführungsformen 13, wobei die Zubereitung, insbesondere ausschließlich, aus einem physiologisch verträglichen Lösungsmittel, vorzugsweise einer physiologischen Kochsalzlösung oder einem physiologisch verträglichen Puffer, insbesondere einer phosphatgepufferten Salzlösung, und der dsRNA besteht.
15. Verwendung nach einer der vorhergehenden Ausführungsformen, wobei die dsRNA in einer physiologisch verträglichen Lösung, insbesondere einem physiologisch verträglichen Puffer oder einer physiologischen Kochsalzlösung, oder von einer micellaren Struktur, vorzugsweise einem Liposom, einem Virus-Kapsid, einem Kapsoid oder einer polymeren Nano- oder Mikrokapsel umschlossen oder an einer polymeren Nano- oder Mikrokapsel gebunden vorliegt.
16. Verwendung nach einer der vorhergehenden Ausführungsformen, wobei die dsRNA oral, mittels Inhalation, Infusion oder Injektion, insbesondere intravenöser, intraperitonealer oder intratumoraler Infusion oder Injektion, verabreicht wird.
17. Verwendung nach einer der vorhergehenden Ausführungsformen, wobei die dsRNA in einer Dosierung von höchstens 5 mg, insbesondere höchstens 2,5 mg, bevorzugt höchstens 200 µg, besonders bevorzugt höchstens 100 µg, vorzugsweise höchstens 50 µg, insbesondere höchstens 25 µg, pro kg Körpergewicht und Tag verwendet wird.
18. Medikament zur gezielten Hemmung der Expression eines vorgegebenen, gegenüber einem Ursprungsgen punktmutierten Zielgens in einer Zelle, wobei das Medikament eine doppelsträngige Ribonukleinsäure (dsRNA) enthält, deren einer Strang S1 einen zum Zielgen komplementären Bereich aufweist, in welchem mindestens ein Nukleotid nicht komplementär zum Zielgen ist und die Anzahl der Nukleotide, welche nicht komplementär zum Ursprungsgen sind, um mindestens eins höher ist als die Anzahl der Nukleotide, welche nicht komplementär zum Zielgen sind.
19. Medikament nach Ausführungsformen 18, wobei das zum Zielgen nicht komplementäre Nukleotid nicht am 3'- oder am 5'-Ende des Bereichs gelegen ist.
20. Medikament nach Ausführungsformen 18 oder 19, wobei das Zielgen gegenüber dem Ursprungsgen eine oder zwei Punktmutationen aufweist.
21. Medikament nach einer der Ausführungsformen 18 bis 20, wobei das Ursprungsgen ein Proto-Onkogen und das Zielgen ein davon abgeleitetes Onkogen ist.
22. Medikament nach einer der Ausführungsformen 18 bis 21, wobei die Zelle eine Tumorzelle ist.
23. Medikament nach einer der Ausführungsformen 18 bis 22, wobei ein Nukleotid des Bereichs nicht komplementär zum Zielgen ist und zwei Nukleotide des Bereichs nicht komplementär zum Ursprungsgen sind.
24. Medikament nach einer der Ausführungsformen 18 bis 23, wobei innerhalb der dsRNA mindestens ein Basenpaar nicht komplementär ist
25. Medikament nach einer der Ausführungsformen 18 bis 24, wobei die dsRNA zumindest an einem Ende der dsRNA einen aus 1 bis 4, insbesondere 2 oder 3, Nukleotiden gebildeten einzelsträngigen Überhang aufweist.
26. Medikament nach Ausführungsformen 25, wobei die dsRNA den Überhang ausschließlich an einem, insbesondere ihrem das 3'-Ende des Strangs S1 aufweisenden, Ende aufweist.
27. Medikament nach Ausführungsformen 26, wobei die dsRNA neben dem Strang S1 einen Strang S2 aufweist und der Strang S1 eine Länge von 23 Nukleotiden, der Strang S2 eine Länge von 21 Nukleotiden und das 3'-Ende des Strangs S1 einen aus zwei Nukleotiden gebildeten einzelsträngigen Überhang aufweist, während das am 5'-Ende des Strangs S1 gelegene Ende der dsRNA glatt ausgebildet ist.
28. Medikament nach einer der Ausführungsformen 18 bis 27, wobei der Strang S1 zum primären oder prozessierten RNA-Transkript des Zielgens komplementär ist.
29. Medikament nach einer der Ausführungsformen 18 bis 28, wobei das Medikament eine Zubereitung aufweist, die zur Inhalation, oralen Aufnahme, Infusion oder Injektion, insbesondere zur intravenösen, intraperitonealen oder intratumoralen Infusion oder Injektion, geeignet ist.
30. Medikament nach Ausführungsformen 29, wobei die Zubereitung, insbesondere ausschließlich, aus einem physiologisch verträglichen Lösungsmittel, vorzugsweise einer physiologischen Kochsalzlösung oder einem physiologisch verträglichen Puffer, insbesondere einer phosphatgepufferten Salzlösung,, und der dsRNA besteht.
31. Medikament nach einer der Ausführungsformen 18 bis 30, wobei die dsRNA in dem Medikament in einer Lösung, insbesondere einem physiologisch verträglichen Puffer oder einer physiologischen Kochsalzlösung, oder von einer micellaren Struktur, vorzugsweise einem Liposom, einem Virus-Kapsid, einem Kapsoid oder einer polymeren Nano- oder Mikrokapsel umschlossen oder an einer polymeren Nano- oder Mikrokapsel gebunden vorliegt.
32. Medikament nach einer der Ausführungsformen 18 bis 31, wobei das Medikament in mindestens einer Verabreichungseinheit vorliegt, welche die dsRNA in einer Menge enthält, die eine Dosierung von höchstens 5 mg, insbesondere höchstens 2,5 mg, bevorzugt höchstens 200 µg, besonders bevorzugt höchstens 100 µg, vorzugsweise höchstens 50 µg, insbesondere höchstens 25 µg, pro kg Körpergewicht und Tag ermöglicht.
33. Verfahren zur gezielten Hemmung der Expression eines vorgegebenen, gegenüber einem Ursprungsgen punktmutierten Zielgens in einer Zelle, wobei eine doppelsträngige Ribonukleinsäure(dsRNA) in die Zelle eingeführt wird und ein Strang S1 der dsRNA einen zum Zielgen komplementären Bereich aufweist, in welchem mindestens ein Nukleotid nicht komplementär zum Zielgen ist und die Anzahl der Nukleotide, welche nicht komplementär zum Ursprungsgen sind, um mindestens eins höher ist als die Anzahl der Nukleotide, welche nicht komplementär zum Zielgen sind.
34. Verfahren nach Ausführungsformen 33, wobei das zum Zielgen nicht komplementäre Nukleotid nicht am 3'- oder am 5'-Ende des Bereichs gelegen ist.
35. Verfahren nach Ausführungsformen 33 oder 34, wobei das Zielgen gegenüber dem Ursprungsgen eine oder zwei Punktmutationen aufweist.
36. Verfahren nach einer der Ausführungsformen 33 bis 35, wobei das Ursprungsgen ein Proto-Onkogen und das Zielgen ein davon abgeleitetes Onkogen ist.
37. Verfahren nach einer der Ausführungsformen 33 bis 36, wobei die Zelle eine Tumorzelle ist.
38. Verfahren nach einer der Ausführungsformen 33 bis 37, wobei ein Nukleotid des Bereichs nicht komplementär zum Zielgen ist und zwei Nukleotide des Bereichs nicht komplementär zum Ursprungsgen sind.
39. Verfahren nach einer der Ausführungsformen 33 bis 38, wobei innerhalb der dsRNA mindestens ein Basenpaar nicht komplementär ist.
40. Verfahren nach einer der Ausführungsformen 33 bis 39, wobei die dsRNA zumindest an einem Ende der dsRNA einen aus 1 bis 4, insbesondere 2 oder 3, Nukleotiden gebildeten einzelsträngigen Überhang aufweist.
41. Verfahren nach Ausführungsformen 40, wobei die dsRNA den Überhang ausschließlich an einem, insbesondere ihrem das 3'-Ende des Strangs S1 aufweisenden, Ende aufweist.
42. Verfahren nach Ausführungsformen 41, wobei die dsRNA neben dem Strang S1 einen Strang S2 aufweist und der Strang S1 eine Länge von 23 Nukleotiden, der Strang S2 eine Länge von 21 Nukleotiden und das 3'-Ende des Strangs S1 einen aus zwei Nukleotiden gebildeten einzelsträngigen Überhang aufweist, während das am 5'-Ende des Strangs S1 gelegene Ende der dsRNA glatt ausgebildet ist.
43. Verfahren nach einer der Ausführungsformen 33 bis 42, wobei der Strang S1 zum primären oder prozessierten RNA-Transkript des Zielgens komplementär ist.
44. Verfahren nach einer der Ausführungsformen 33 bis 43, wobei die dsRNA in einer Lösung, insbesondere einem physiologisch verträglichen Puffer oder einer physiologischen Kochsalzlösung, oder von einer micellaren Struktur, vorzugsweise einem Liposom, einem Virus-Kapsid, einem Kapsoid oder einer polymeren Nano- oder Mikrokapsel umschlossen oder an einer polymeren Nano- oder Mikrokapsel gebunden vorliegt.

## Patentansprüche

1. Verwendung einer doppelsträngigen Ribonukleinsäure (dsRNA) zur gezielten Hemmung der Expression eines vorgegebenen, gegenüber einem Ursprungsgen punktmutierten Zielgens in einer Zelle, wobei ein Strang S1 der dsRNA einen zum Zielgen komplementären Bereich aufweist, in welchem mindestens ein Nukleotid nicht komplementär zum Zielgen ist und die Anzahl der Nukleotide, welche nicht komplementär zum Ursprungsgen sind, um mindestens eins höher ist als die Anzahl der Nukleotide, welche nicht komplementär zum Zielgen sind.

2. Verwendung einer doppelsträngigen Ribonukleinsäure (dsRNA) zur Herstellung eines Medikaments zur gezielten Hemmung der Expression eines vorgegebenen, gegenüber einem Ursprungsgen punktmutierten Zielgens in einer Zelle, wobei ein Strang S1 der dsRNA einen zum Zielgen komplementären Bereich aufweist, in welchem mindestens ein Nukleotid nicht komplementär zum Zielgen ist und die Anzahl der Nukleotide, welche nicht komplementär zum Ursprungsgen sind, um mindestens eins höher ist als die Anzahl der Nukleotide, welche nicht komplementär zum Zielgen sind.

3. Verwendung nach einem der vorhergehenden Ansprüche, wobei
(a) die dsRNA in einer Zubereitung vorliegt, die zur Inhalation, oralen Aufnahme, Infusion oder Injektion, insbesondere zur intravenösen, intraperitonealen oder intratumoralen Infusion oder Injektion, geeignet ist;
(b) die dsRNA in einer physiologisch verträglichen Lösung, insbesondere einem physiologisch verträglichen Puffer oder einer physiologischen Kochsalzlösung, oder von einer micellaren Struktur, vorzugsweise einem Liposom, einem Virus-Kapsid, einem Kapsoid oder einer polymeren Nano- oder Mikrokapsel umschlossen oder an einer polymeren Nano- oder Mikrokapsel gebunden vorliegt;
(c) die dsRNA oral, mittels Inhalation, Infusion oder Injektion, insbesondere intravenöser, intraperitonealer oder intratumoraler Infusion oder Injektion, verabreicht wird; und/oder
(d) die dsRNA in einer Dosierung von höchstens 5 mg, insbesondere höchstens 2,5 mg, bevorzugt höchstens 200 µg, besonders bevorzugt höchstens 100 µg, vorzugsweise höchstens 50 µg, insbesondere höchstens 25 µg, pro kg Körpergewicht und Tag verwendet wird.

4. Medikament zur gezielten Hemmung der Expression eines vorgegebenen, gegenüber einem Ursprungsgen punktmutierten Zielgens in einer Zelle, wobei das Medikament eine doppelsträngige Ribonukleinsäure (dsRNA) enthält, deren einer Strang S1 einen zum Zielgen komplementären Bereich aufweist, in welchem mindestens ein Nukleotid nicht komplementär zum Zielgen ist und die Anzahl der Nukleotide, welche nicht komplementär zum Ursprungsgen sind, um mindestens eins höher ist als die Anzahl der Nukleotide, welche nicht komplementär zum Zielgen sind.

5. Medikament nach Anspruch 4, wobei
(a) das Medikament eine Zubereitung aufweist, die zur Inhalation, oralen Aufnahme, Infusion oder Injektion, insbesondere zur intravenösen, intraperitonealen oder intratumoralen Infusion oder Injektion, geeignet ist;
(b) die dsRNA in dem Medikament in einer Lösung, insbesondere einem physiologisch verträglichen Puffer oder einer physiologischen Kochsalzlösung, oder von einer micellaren Struktur, vorzugsweise einem Liposom, einem Virus-Kapsid, einem Kapsoid oder einer polymeren Nano- oder Mikrokapsel umschlossen oder an einer polymeren Nano- oder Mikrokapsel gebunden vorliegt; und/oder
(c) das Medikament in mindestens einer Verabreichungseinheit vorliegt, welche die dsRNA in einer Menge enthält, die eine Dosierung von höchstens 5 mg, insbesondere höchstens 2,5 mg, bevorzugt höchstens 200 µg, besonders bevorzugt höchstens 100 µg, vorzugsweise höchstens 50 µg, insbesondere höchstens 25 µg, pro kg Körpergewicht und Tag ermöglicht.

6. Medikament nach Anspruch 5(a), wobei die Zubereitung, insbesondere ausschließlich, aus einem physiologisch verträglichen Lösungsmittel, vorzugsweise einer physiologischen Kochsalzlösung oder einem physiologisch verträglichen Puffer, insbesondere einer phosphatgepufferten Salzlösung, und der dsRNA besteht.

7. Verfahren zur gezielten Hemmung der Expression eines vorgegebenen, gegenüber einem Ursprungsgen punktmutierten Zielgens in einer Zelle, wobei eine doppelsträngige Ribonukleinsäure(dsRNA) in die Zelle eingeführt wird und ein Strang S1 der dsRNA einen zum Zielgen komplementären Bereich aufweist, in welchem mindestens ein Nukleotid nicht komplementär zum Zielgen ist und die Anzahl der Nukleotide, welche nicht komplementär zum Ursprungsgen sind, um mindestens eins höher ist als die Anzahl der Nukleotide, welche nicht komplementär zum Zielgen sind.

8. Verwendung nach einem der Ansprüche 1 bis 3, Medikament nach einem der Ansprüche 4 bis 6, oder Verfahren nach Anspruch 7, wobei das zum Zielgen nicht komplementäre Nukleotid nicht am 3'- oder am 5'-Ende des Bereichs gelegen ist.

9. Verwendung nach einem der Ansprüche 1 bis 3, Medikament nach einem der Ansprüche 4 bis 6, oder Verfahren nach Anspruch 7 oder 8, wobei das Zielgen gegenüber dem Ursprungsgen eine oder zwei Punktmutationen aufweist.

10. Verwendung nach einem der Ansprüche 1 bis 3, Medikament nach einem der Ansprüche 4 bis 6, oder Verfahren nach einem der Ansprüche 7 bis 9, wobei das Ursprungsgen ein Proto-Onkogen und das Zielgen ein davon abgeleitetes Onkogen ist.

11. Verwendung nach einem der Ansprüche 1 bis 3, Medikament nach einem der Ansprüche 4 bis 6, oder Verfahren nach einem der Ansprüche 7 bis 10, wobei die Zelle eine Tumorzelle ist.

12. Verwendung nach einem der Ansprüche 1 bis 3, Medikament nach einem der Ansprüche 4 bis 6, oder Verfahren nach einem der Ansprüche 7 bis 11, wobei ein Nukleotid des Bereichs nicht komplementär zum Zielgen ist und zwei Nukleotide des Bereichs nicht komplementär zum Ursprungsgen sind.

13. Verwendung nach einem der Ansprüche 1 bis 3, Medikament nach einem der Ansprüche 4 bis 6, oder Verfahren nach einem der Ansprüche 7 bis 12, wobei die dsRNA zumindest an einem Ende der dsRNA einen aus 1 bis 4, insbesondere 2 oder 3, Nukleotiden gebildeten einzelsträngigen Überhang aufweist.

14. Verwendung, Medikament, oder Verfahren nach Anspruch 13, wobei die dsRNA den Überhang ausschließlich an einem, insbesondere ihrem das 3'-Ende des Strangs S1 aufweisenden, Ende aufweist.

15. Verwendung, Medikament, oder Verfahren nach Anspruch 14, wobei die dsRNA neben dem Strang S1 einen Strang S2 aufweist und der Strang S1 eine Länge von 23 Nukleotiden, der Strang S2 eine Länge von 21 Nukleotiden und das 3'-Ende des Strangs S1 einen aus zwei Nukleotiden gebildeten einzelsträngigen Überhang aufweist, während das am 5'-Ende des Strangs S1 gelegene Ende der dsRNA glatt ausgebildet ist.
